# EUROPEAN PATENT APPLICATION

(11) **EP 1 661 556 A1**
(43) Date of publication of application: **31.05.2006**
(21) Application number: 04771851.5
(22) Date of filing: 19.08.2004
(51) Int. Cl.: A61K 9/08, A61K 9/16, A61K 47/20, A61K 47/22, A61K 47/32, A61K 47/34, A61K 47/36, A61K 47/40

(54) **MEDICINAL PREPARATION HAVING IMPROVED DISSOLUTION PROPERTIES**

(30) Priority: 20.08.2003 JP 2003296396
(71) Applicant: Ajinomoto Co., Inc., Tokyo 104-8315 (JP)
(72) Inventor: MAKINO, Chisato, AJINOMOTO CO., INC., Kawasaki-shi, Kanagawa 2108681 (JP)
(74) Representative: Nicholls, Kathryn Margaret
(86) International application number: PCT/JP2004/011890
(87) International publication number: WO 2005/018607

(57) **Abstract**

The present invention provides a method for producing a pharmaceutical preparation having an improved solubility, which comprises the steps of forming a micelle by dissolving a poorly-soluble drug having the ability to form the micelle into water; and fixing the micelle structure which is formed with the poorly-soluble drug, by using a compound(s) which fixes the micelle structure. This production method preferably further comprises the step of adjusting the pH back to neutral and/or cooling down the temperature to room temperature after fixing the micelle structure. The poorly-soluble drug which is the subject of the above method is such as those forming a micelle in water only when heated and/or in the presence of an acid(s) or alkali(s). According to this production method, it can produce a pharmaceutical preparation having an improved solubility, which makes possible to accomplish the improvement of oral absorbability of a poorly-soluble drug(s).

## Description

### Technical Field of the Invention

The present invention relates to preparation techniques such as solubilization and immediate-releasing of poorly-soluble drug, and solubilized or immediate-released pharmaceutical preparations.

In recent years, many of compounds found in the drug development are poorly-soluble drugs which are poorly soluble in water. In case of developing oral preparations, it has been seen as problems that, when the poorly-soluble drugs are orally administered, the oral absorption rate declines or varies because of their low solubility.

In order to solve the above problems, various studies have been conducted on methods for preparing pharmaceutical products. For example, methods have been reported such as (1) the method that a poorly-soluble drug(s) and a water-soluble polymer(s) are dissolved into an organic solvent(s), and then the organic solvent(s) is removed to obtain a solid dispersion(s) (as indicated in Patent Literature 1 and Non-Patent Literature 1, for instance); (2) the method that particles of a poorly-soluble drug(s) are finely ground to improve the speed of dissolution (as indicated in Patent Literature 2, for instance); and (3) the method that a surfactant(s) is used for solubilization (as indicated in Patent Literature 3, for example).

However, since the organic solvent(s) is voluminously used in the method (1), its effect on the environment is problematic when producing. In addition to it, it is also difficult to completely remove the solvent(s). It is known that the method (2) requires the longer processing time and therefore, its production cost is problematic. As for the solubilizing method (3), the technique for efficiently solubilizing a poorly-soluble drug(s) has not yet been reported.
[Patent Literature 1] WO 02/34254
[Patent Literature 2] WO 00/57881
[Patent Literature 3] WO97/41894
[Non-Patent Literature 1] "Keikoutouyoseizai no Sekkei to Hyouka (Design and Evaluation on Orally Administered Drugs)", Mitsuru Hashida, Ed., Yakugyo-Jiho Sha, p. 178, 1995

### Disclosure of the Invention

The object of the present invention is to provide a method for producing a pharmaceutical preparation(s) having an improved solubility, which makes it possible to accomplish the improvement of oral absorbability of a poorly-soluble drug(s).

The further object of the present invention is to provide the method for producing the pharmaceutical preparation(s) having an improved solubility, which makes it possible to solubilize or immediate-release the poorly-soluble drug(s).

The additional object of the present invention is to provide the pharmaceutical preparation(s) having an improved solubility, which makes it possible to accomplish the improvement of oral absorbability of the poorly-soluble drug(s).

The present invention has been completed based on the finding that, in case of poorly-soluble drugs such as those forming a micelle (molecular assembly) in water only when heated and/or in the presence of an acid(s) or alkali(s), the structure of the molecular assembly is fixed when a surfactant(s) and the like is added to the molecular assembly system; then, even if the pH is further back to neutral or the temperature is cooled down to room temperature, a clear liquid containing a solubilized drug is obtained; further, the oral absorbability of said liquid is improved as compared with a powdery drug(s); and then, the improvement effect of said oral absorbability is maintained even if the liquid containing a solubilized drug is prepared in its solid form.

Namely, the present invention provides a method for producing a pharmaceutical preparation having an improved solubility, which comprises the steps of forming a micelle by dissolving a poorly-soluble drug having the ability to form the micelle into water; and fixing the micelle structure which is formed with the poorly-soluble drug, by using a compound(s) which fixes the micelle structure.

The present invention also provides a pharmaceutical preparation produced by the above production method.

Additionally, the present invention provides a pharmaceutical composition containing the pharmaceutical preparation which is prepared by fixing the micelle structure of the poorly-soluble drug with the compound which fixes the micelle structure.

### Best Mode for Carrying out the Invention

The poorly-soluble drugs which are the subjects of the present invention are not particularly limited as long as they are poorly soluble in water (for example, the solubility in 1000g of water at 25°C is 1g or less) but form a micelle (molecular assembly) in water only when heated and/or in the presence of an acid(s) (preferably a strong acid(s)) or alkali(s) (preferably a strong alkali(s)) to form a clear solution. Such drugs include a compound(s) having at least one dissociating group(s) in the molecule and a hydrophobic group(s), if necessary.

Specifically, examples of such drugs include (4-(5H-dibenzo[a,d] cyclohepten-5-ylidene)-1-[3-[4-sulfamoylphenyl]-2(E)-propenyl]-piperidine, monohydro-chloride) (hereinafter referred to as "AP-1067") having the following structural formula and 5-(((S)-2,2-climethyl -cyclopropancarbonyl)amino)-2-(4-(((S)-2,2-dimethylcyclopropancarbonyl) amino)phenoxy)pyridine (hereinafter referred to as "APC0576") (Takehana et, al. Biochem. Biophys. Res. Commun. 293 (2002) 945-952). Further, they include compounds having a cyproheptadine moiety such as cyproheptadine; and compounds having other moieties such as glycyrrhizinic acid, tocopherol succinic acid, hydrocortisone succinic acid, prednisolone succinic acid, perphenazine, dantrolene sodium, tinidazole, dehydrocholic acid, lidocaine, tolazamide, trepibutone, naproxen, miconazole, tocopherol nicotinic acid, haloperidol, proglumide, probenecid, fenbufen, pranoprofen, flurbiprofen, nateglinide, the compounds of Chemical Formulae 3 or 5 described in Japanese Patent Unexamined Publication No. Hei 11-116502, and the compounds of Chemical Formulae 2, 5, 6, 7, 8 or 9 described in Japanese Patent Unexamined Publication No. Hei 7-109218.

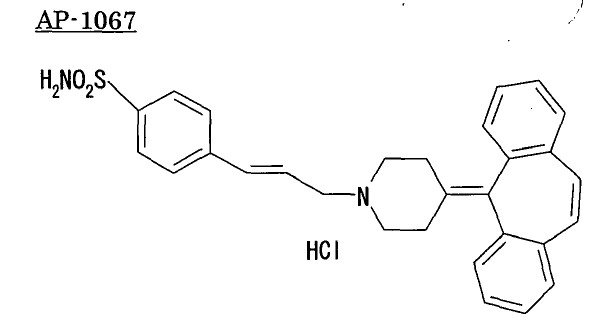

In the present invention, examples of the compounds which fix the micelle structure include surfactants and water-soluble polymers.

Examples of the surfactants include one kind or mixtures of two or more kinds of anionic surfactants, cationic surfactants and nonionic surfactants.

Examples of anionic surfactants include sodium lauryl sulfate and dicetyl phosphate, and they are not particularly limited as long as they are pharmaceutically acceptable.

Examples of cationic surfactants include cetylpyridinium chloride, and they are not particularly limited as long as they are pharmaceutically acceptable.

Examples of the nonionic surfactants include polyoxyethylene-type nonionic surfactants having polyoxyethylene as a hydrophilic group(s), such as polysorbates, polyoxyethylene hydrogenated caster oils, and Polyoxyl 40 Stearate. As examples of the other nonionic surfactants, sucrose fatty acid esters can be used, and they are not particularly limited as long as they are pharmaceutically acceptable.

Water-soluble polymers include one kind or mixtures of two or more kinds of polysaccharide derivatives, polyacrylic acid derivatives, polyoxyethylene, polyvinylpyrrolidone derivatives, polyvinylalcohol, and cyclodextrins.

Examples of polysaccharides include cellulose derivatives such as methylcellulose, hydroxypropyl methylcellulose and hydroxypropyl cellulose particularly, and they are not particularly limited as long as they are pharmaceutically acceptable.

Examples of polyvinylpyrrolidone derivatives include polyvinylpyrrolidone and 1-vinyl-2-pyrrolidone-vinyl acetate copolymers, and they are not particularly limited as long as they are pharmaceutically acceptable.

Examples of cyclodextrins include α -cyclodextrin, β -cydodextrin, γ - cyclodextrin, hydroxypropyl-β -cyclodextrin, and sulfobutylether- β -cyclodextrin.

In the present invention, firstly, a poorly-soluble drug(s) having the ability to form a micelle is dissolved into water containing an acid(s) or alkali(s) to form the micelle. In this connection, acids are preferably strong acids (and more preferably inorganic acids) such as hydrochloric acid, sulfuric acid, and phosphoric acid. Alkalis are preferably strong alkalis (and more preferably inorganic alkali compounds) such as sodium hydroxide and potassium hydroxide. Further, the pH of an aqueous solution containing an acid(s) is preferably 3 or lower, and that of an aqueous solution containing an alkali(s) is preferably 9 or higher. At any rate, it is preferable that the pH is adjusted to become suitable for the micelle formation in water by a poorly-soluble drug.

Besides, it is preferable that an acidic aqueous solution or alkaline aqueous solution is heated to 35 to 100°C and more preferably 40 to 60°C, and a poorly-soluble drug is dissolved thereinto to form the micelle. However, it is possible to conduct the treatment at room temperature without heating.

Further, it is also possible to heat water up to the above temperature without adding an acid(s) or alkali(s) and dissolve a poorly-soluble drug thereinto in order to form the micelle in water.

Thus, the micelle is formed by dissolving a poorly-soluble drug into water. After that, in the present invention, a compound(s) which fixes the micelle structure fixes the micelle structure formed by the poorly-soluble drug.

In the present invention, fixing the micelle structure means that a poorly-soluble drug(s) such as those forming a micelle in water only when heated and/or in the presence of an acid(s) or alkah(s), as mentioned above forms the micelle in the presence of an acid(s) or alkali(s) and/or by heating to obtain a clear solution; and the once formed micelle structure is stabilized by adding a certain compound(s) thereto or the like; and then the micelle structure continues to be maintained even if the pH is back to neutral or the temperature is cooled down to room temperature.

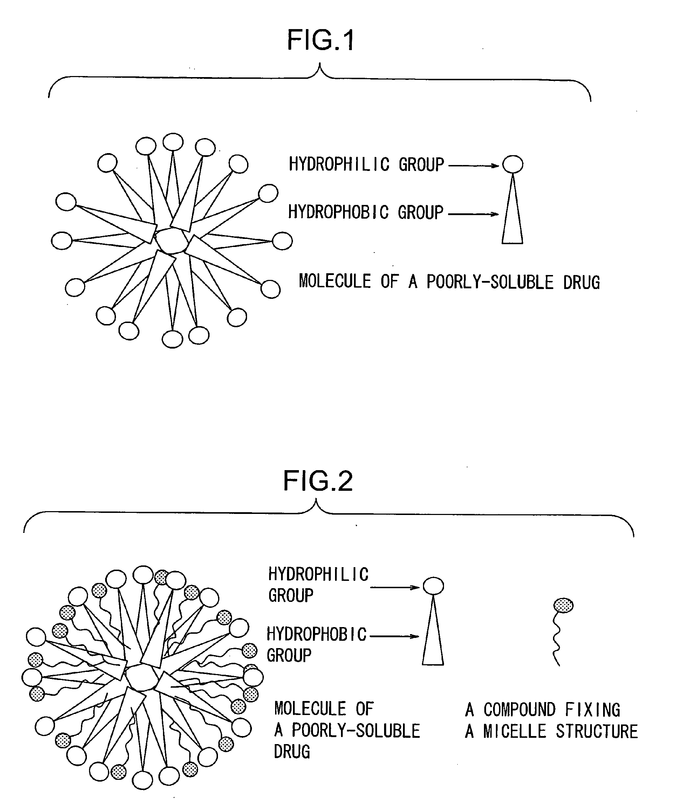

It is thought that the following phenomenon occurs when a poorly-soluble drug(s) is dissolved as mentioned above. Namely, it is thought that the dissociating groups dissociate and the hydrophobic groups assemble (Fig. 1) in the poorly-soluble drug(s) which has formed the micelle structure, for example, in the presence of alkali(s) or by heating. When the pH of the liquid is back to neutral, the dissociation degree of the dissociating groups is inhibited, and the hydrophobic groups are bared by the inhibition of repulsion between the dissociating groups, and they come into contact with water and precipitates. On the other hand, when a compound(s) such as a surfactant(s) which fixes a micelle is added to the liquid under the condition that the micelle has once been formed, hydrophobic parts of the surfactant(s) penetrate into spaces (hydrophobic parts) of the micelle formed by the poorly-soluble drug(s) (Fig. 2). Thus, the micelle structure is stabilized and can continue to be maintained even if the pH is back to neutral or the temperature is cooled down to room temperature.

The order of addition in the micelle solidification may be, for example, either of: a method comprising the steps of dissolving a poorly-soluble drug into water to form a micelle, and adding a compound(s) which fixes the micelle structure to fix the micelle structure formed by the poorly-soluble drug; or a method comprising the steps of dissolving a poorly-soluble drug into water to form a micelle in the presence of a compound(s) which fixes the micelle structure, and fixing the micelle structure formed by the poorly-soluble drug.

The amount of addition of a compound(s) which fixes the micelle structure can be optional as long as the micelle structure of the poorly-soluble drug is stable in water and not destroyed even if the pH becomes neutral or the temperature becomes room temperature. For instance, the compound(s) which fixes the micelle structure is preferably used in the amount of 0.01mg to 20g per 1g of the poorly-soluble drug.

In the present invention, after fixing the micelle structure formed by the poorly-soluble drug, it can be directly used or, in case that the solution is made acidic or alkaline by addition of an acid(s) or alkali(s), it is preferably made neutral by addition of an alkali(s) or acid(s). Similarly, in case that the solution is heated, it is preferably cooled down to room temperature.

In the present invention, a pharmaceutical preparation(s) thus prepared can be directly used or further prepared in its solid form by removing water with the ordinary methods. The process for preparing such solid form can be conducted by wet granulation such as fluidized bed granulation, high-speed mixing/granulation, spray-dry and freeze-dry. In this connection, the wet granulation, for instance, includes a method comprising the steps of spraying a liquid pharmaceutical preparation(s) on pharmaceutically acceptable particulates or powders such as the following inactive diluent(s), swelling agent(s), sweetening agent(s) and the like to granulate them.

Pharmaceutical compositions of the present invention can contain known auxiliary substances like excipients such as lactose, sucrose, and corn starch; binders such as hydroxypropyl cellulose, hydroxypropyl methylcellulose, and macrogol; disintegrating agents such as low-substituted hydroxypropyl cellulose, crystalline cellulose, croscarmellose sodium, and carboxymethyl starch; sweetening agents such as saccharin, aspartame, and acesulfame-K; lubricants such as magnesium stearate and talc; and surfactants such as alginic acid, sodium lauryl sulfate and Polysorbate 80, in addition to the above pharmaceutical preparation(s).

The dosage forms of such pharmaceutical compositions can be, for example, tablets, powders, pills, granules, capsules, solutions, sugar-coated agents, or syrups.

Next, Examples will further illustrate the present invention.

### Referential Example 1 Confirming the formation of a micelle (molecular assembly)

Alkali aqueous liquid (pH=13) containing solubilized AP-1067 (4-(5H-dibenzo[a,d] cyclohepten-5-ylidene)-1-[3-[4-sulfamoylphenyl] -2(E)-propenyl]-piperidine, monohydro-chloride) having various concentrations were prepared, and their surface tensions were determined. As a result, it was clarified that higher the concentration of AP-1067 was, lower the surface tension became, and the tension became constant in the specified concentration or above. Namely, it was thought that the critical micelle concentration existed in the liquid and a molecular assembly (a micelle) was formed.

### Example 1 Solubilization of AP-1067

About 10mg of AP-1067 was dispersed into 1mL of water. An aqueous solution of sodium hydroxide was added thereto to adjust the solution to pH=13 or above. The suspension was heated up to 45°C to obtain a clear alkali aqueous liquid containing solubilized AP-1067 wherein a micelle of AP-1067 was formed.

### (AP-1067 concentration: about10mg/mL)

40mg of sodium dodecyl sulfate (SDS) was added to the alkali aqueous liquid to fix the formed micelle structure. Then, an aqueous solution of hydrochloric acid was added dropwise thereto and its pH was adjusted to neutral to obtain a clear neutral liquid containing slubilized AP-1067 wherein the micelle of AP-1067 existed in the fixed manner. The concentration of AP-1067 at that time was 9.1mg/mL.

Since the solubility of AP-1067 in water at room temperature is about 0.03mg/mL, the present method of solubilization improved its solubility to about 300 times.

### Referential Example 2 Confirming the solidification of the micelle (molecular assembly) formation

The liquid containing solubilized AP-1067 was prepared by the same method as that of Example 1 using deuterated water, deuterated sodium hydroxide, and deuterium chloride, and NMR (NOESY) determination was conducted.

From the obtained data, the cross peak was seen between methylene proton of the central part in a long-chain alkyl chain of SDS and proton of the tricyclic part of AP-1067 and it was confirmed that the both protons exist close each other. Further, when the pH of the liquid was adjusted to neutral, the cross peak was seen between methylene proton of the central part and terminal methyl proton in a long alkyl chain of SDS and proton of the tricyclic part of AP-1067 and it was confirmed that the both protons exist close each other. Thus, it was thought that AP-1067 and SDS, which was a surfactant, existed in the fixed micelle form in the liquid as shown in Fig. 2.

### Example 2 Solubilization of AP-1067

About 10mg of AP-1067 was dispersed into 1mL of water. An aqueous solution of sodium hydroxide was added thereto to adjust the solution to pH=13 or above. The suspension was heated up to 45°C to obtain a clear alkali aqueous solution of AP-1067 wherein a micelle of AP-1067 was formed. (AP-1067 concentration: about 10mg/mL)

100mg of Polysorbate 80 was added to the alkali aqueous solution to fix the formed micelle structure. Then, an aqueous solution of hydrochloric acid was added dropwise thereto and its pH was adjusted to neutral to obtain a clear neutral liquid containing solubilized AP-1067 wherein the micelle of AP-1067 existed in the fixed manner. The concentration of AP-1067 at that time was 10.3mg/mL.

Since the solubility of AP-1067 in water at room temperature is about 0.03mg/mL, the present method of solubilization improved its solubility to about 300 times.

### Comparative Example 1

10mg of AP-1067 and 40mg of SDS were dispersed into 1mL of water. The suspension was heated up to 45°C, and the ultrasonic treatment was conducted for 1.5 hours. However, a clear liquid of AP-1067 could not be obtained.

### Comparative Example 2

10mg of AP-1067 and 100mg of Polysorbate 80 were dispersed into 1mL of water. The suspension was heated up to 45°C, and the ultrasonic treatment was conducted for 1.5 hours. However, a clear liquid of AP-1067 could not be obtained.

### Example 3 Solubilization of APC0576

10mg of APC0576 and 83mg of cetylpyridinium chloride monohydrate were dispersed and dissolved into 1mL of an aqueous solution of 1.5mol/L hydrochloric acid. The suspension was heated up to 50°C, and the ultrasonic treatment was conducted thereto to obtain a clear acidic aqueous liquid containing solubilized APC0576 wherein a micelle of the poorly-soluble drug APC0576 existed in the fixed manner. (APC057G concentration: about10mg/mL)

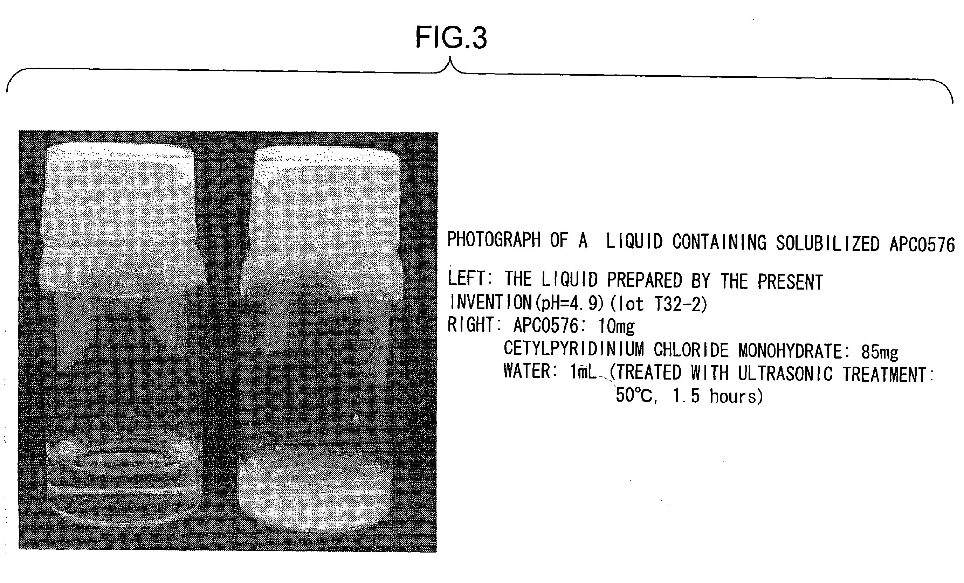

An aqueous solution of 10mol/L sodium hydroxide was added dropwise to the acidic aqueous liquid to adjust its pH to nearly neutral to obtain about 7mg/mL of a clear liquid containing solubilized APC0576 wherein the micelle of APC0576 existed in the fixed manner. (see Fig. 3)

Since the solubility of APC0576 in water at room temperature is about 0.0016mg/mL, the present method of solubilization improved its solubility to about 4400 times.

### Comparative Example 3

10mg of APC0576 and 85mg of cetylpyridinium chloride monohydrate were dissolved into 1mL of water. The suspension was heated up to 50°C, and the ultrasonic treatment was conducted for 1.5 hours. However, a clear solubilized solution of APC0576 could not be obtained. (see Fig. 3)

### Example 4 Preparing the solid dosage form of the liquid containing solubilized AP-1067

250g of partly pregelatinized starch (Asahi Kasei Chemicals Corporation, PCS) was set to High Speed Mixer Mini produced by Fukae-Kogyo KK. Then, 338.9g of a liquid containing solubilized AP-1067 obtained by the same method as that of Example 1 (the concentration of AP-1067: 29.5mg/mL, the concentration of SDS: 118mg/mL) was added dropwise, and the high speed mixing/granulation was conducted. The obtained granulated substance was dried on the plate and screened through a sieve to obtain a granulated pharmaceutical preparation.

### Example 5 Evaluation on dissolution profiles of the liquid containing solubilized AP-1067 and the granules prepared with the liquid

The dissolution profiles of the poorly-soluble drug AP-1067 in each of the liquid containing solubilized AP-1067 obtained in Example 1 and the granulated pharmaceutical preparation (mentioned as granules prepared with the liquid containing solubilized drug in Figs.) obtained in Example 4 was evaluated in accordance with the puddle method (50rpm, an aqueous solution of 0.1w/v% Polysorbate 80: 900mL, AP-1067: 10mg/vessel). The liquid containing solubilized drug and the granulated pharmaceutical preparation were evaluated as n=3, and others were evaluated as n=1.

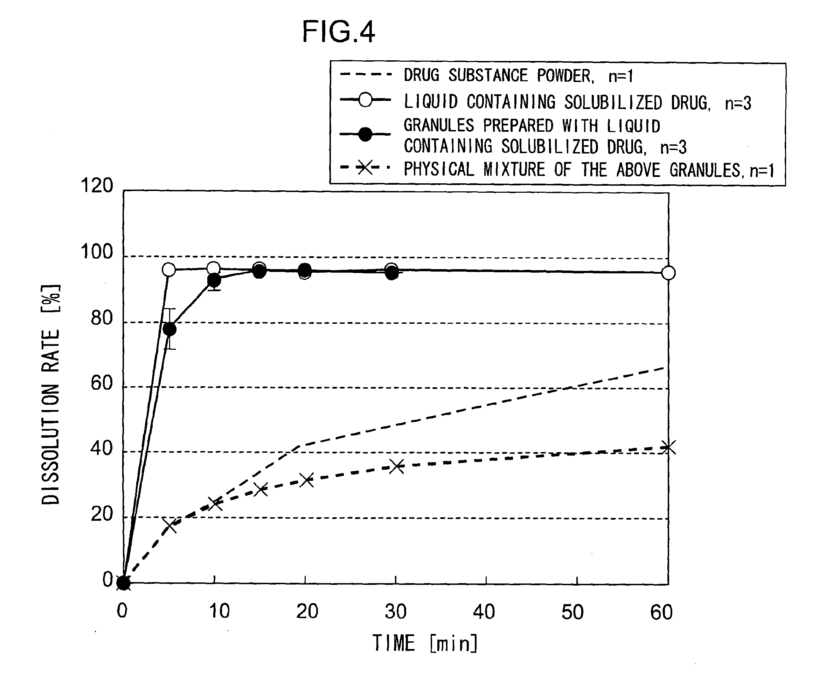

The results are shown in Fig. 4. The dissolution rate of the liquid containing solubilized AP-1067 and the granulated pharmaceutical preparation of the present invention were significantly improved compared with AP-1067 powder.

### Example 6 Evaluation on oral absorbability in dogs

When AP-1067 preparations were orally administered to beagle dogs, the profiles of its concentration in blood plasma (administration after fasting, AP-1067: 3mg/kg, n=3, average ± SE) was examined on each of the liquid containing solubilized AP-1067 obtained in Example 1 and the granulated pharmaceutical preparation (mentioned as granules absorbing the solubilized solution in Figs.) obtained in Example 4.

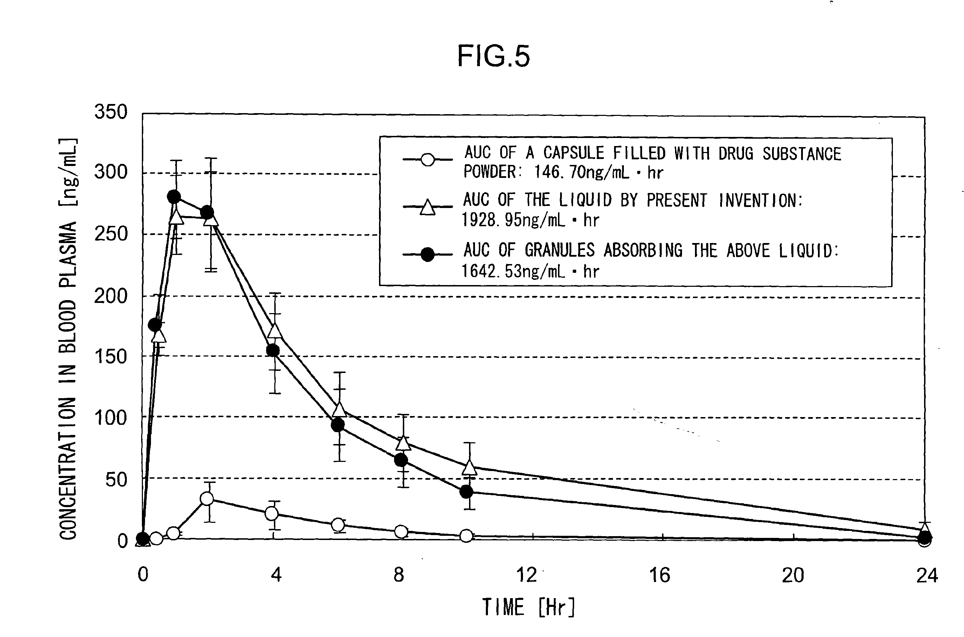

The results are shown in Fig. 5 and Table 1. It is clarified from them that, compared with capsules filled with A-P-1067 drug powder, AUC and Cmax are significantly improved by solubilizing AP-1067 in accordance with the present invention. It is also clarified that the effect is hardly decreased even though the liquid containing solubilized drug is prepared to its solid form.

**Table 1: Improvement effect of oral absorbability of AP-1067 devised preparation**

| AP-1067 preparation | AUC ratio | Cmax ratio |
|---|---|---|
| Particles prepared with the liquid containing solubilized drug | 11.2 | 9.6 |
| The liquid containing solubilized drug | 13.1 | 8.7 |

According to the present invention, it provides a pharmaceutical preparation(s) wherein the solubility of a poorly-soluble drug(s) and the oral absorbability thereof are improved, and its effective production method.

### Brief Description of the Drawings

Fig. 1 shows the status wherein the poorly-soluble drug forms the micelle.
Fig. 2 shows the status wherein the micelle of the poorly-soluble drug is fixed by adding the compound which fixes the micelle structure to the poorly-soluble drug forming the micelle.
Fig. 3 shows the photograph indicating the status of the solubilized solution of the poorly-soluble drug APC0576 prepared in accordance with Example 3, and that of the suspension of the poorly-soluble drug APC0576 prepared in accordance with Comparative Example 3.
Fig. 4 shows the dissolution profiles of the liquid containing solubilized AP-1067 of the present invention and that of the poorly-soluble drug AP-1067 in the form of the granulated preparation.
Fig. 5 shows the profiles of the concentration of AP-1067 in blood plasma when the liquid containing solubilized AP-1067 and the granulated preparation of the present invention were orally administered to beagle dogs.

## Claims

1. A method for producing a pharmaceutical preparation having an improved solubility, which comprises the steps of forming a micelle by dissolving a poorly-soluble drug having the ability to form the micelle in water; and fixing the micelle structure which is formed with the poorly-soluble drug, by using a compound(s) which fixes the micelle structure.

2. The method of claim 1, wherein the poorly-soluble drug forms the micelle in water only when heated and/or in the presence of an acid(s) or alkali(s).

3. The method of claim 1 or 2, which further comprises the step of adjusting the pH back to neutral and/or cooling down the temperature to room temperature after fixing the micelle structure.

4. The method of any one of claims 1 to 3, wherein the compound which fixes the micelle structure is a surfactant and/or a water-soluble polymer.

5. The method of claim 4, wherein the surfactant and/or the water-soluble polymer are at least one kind of the compounds selected from anionic surfactants, cationic surfactants, nonionic surfactants, polysaccharides, polyacrylic acids, polyethers, and cyclodextrins.

6. The method of claim 4, wherein the surfactant is a polyoxyethylene-type nonionic surfactant.

7. The method of claim 4, wherein the surfactant and/or the water-soluble polymer are at least one kind of the compounds selected from sodium lauryl sulfate, cetylpyridinium chloride, Polysorbate 80, α -cyclodextrin, β -cyclodextrin, γ *-* cyclodextrin, hydroxypropyl- β -cyclodextrin, and sulfobutylether- β -cyclodextrin.

8. The method of any one of claims 1 to 7, wherein the poorly-soluble drug is dissolved into an acidic or alkaline aqueous solution to form the micelle.

9. The method of any one of claims 1 to 7, wherein the poorly-soluble drug is dissolved into a heated acidic or alkaline aqueous solution to form the micelle.

10. The method of any one of claims 1 to 9, which further comprises the step of preparation to the solid form after fixing the micelle structure which is formed with the poorly-soluble drug.

11. The method of claim 10, wherein the process of the preparation to the solid form is conducted by wet granulation.

12. The method of claim 10, wherein the process of the preparation to the solid form is conducted by fluidized bed granulation, high-speed mixing/granulation, spray-dry or freeze-dry.

13. A pharmaceutical preparation which is produced by the method according to any one of claims 1 to 12.

14. A pharmaceutical composition containing a pharmaceutical preparation which is prepared by fixing the micelle structure of a poorly-soluble drug using a compound(s) which fixes the micelle structure.

15. A pharmaceutical composition containing the pharmaceutical preparation which is produced by the method according to any one of claims 1 to 12.
